# EUROPEAN PATENT SPECIFICATION

(11) **EP 3 747 371 B1**
(45) Date of publication and mention of the grant of the patent: **27.11.2024**
(21) Application number: 20177661.4
(22) Date of filing: 01.06.2020
(51) Int. Cl.: A61B 17/02, A61C 5/82, A61C 5/90, A61B 13/00

(54) **DENTAL RETRACTION DEVICE**
ZAHNÄRZTLICHE RETRAKTIONSVORRICHTUNG
DISPOSITIF DE RÉTRACTION DENTAIRE

(30) Priority: 03.06.2019 US 201916430168
(43) Date of publication of application: 09.12.2020
(60) Divisional application: 24207557.0
(73) Proprietor: Ultradent Products, Inc., South Jordan, UT 84095 (US)
(72) Inventor: JESSOP, Neil, South Jordan, UT 84095 (US)
(74) Representative: Hepworth Browne

(56) References cited:
- EP-A1- 2 967 304
- EP-B1- 2 967 304
- WO-A1-2018/089862
- KR-A- 20080 036 728
- US-A1- 2013 288 196

## Description

### BACKGROUND OF THE INVENTION

### Field of the Invention

The present invention generally relates to dental and oral components such as dental retraction devices, in particular, some embodiments of the present disclosure relate to tongue retractors for dental retraction devices.

### Description of Related Art

Dental retraction devices may be implemented during dental procedures. The dental retraction devices may be positioned in a mouth of a user or a patient to separate oral tissue such as cheeks and lips from teeth of the user. Dental retraction devices may include a feature or component that positions the tongue relative to remaining portions of the dental retraction device. For example, dental retraction devices may include a tongue guard placed over the tongue. However, the existing dental retraction devices position the tongue or retain the tongue in an uncomfortable position, may retain the tongue in a position that does not adequately separate the tongue from the teeth, or may trigger a gag reflex of the user.

Korean document KR20080036728 discloses a lip and tongue retractor for dental treatment, wherein the retractor is configured to be mounted inside a user's mouth simply and to prevent a user's tongue from being moved to protect the user's tongue. The lip and tongue retractor includes a first shell, a second shell, a link and a tongue holder. The first shell and the second shell come in contact with a user's cheek. The link connects the first shell and the second shell. The tongue holder includes a tongue depressed portion, which is fixed by being inserted into the link.

The subject matter claimed herein is not limited to embodiments that solve any disadvantages or that operate only in environments such as those described. Rather, this background is only provided to illustrate one example technology area where some embodiments described herein may be practiced.

### BRIEF SUMMARY OF EMBODIMENTS OF THE INVENTION

A need therefore exists for transpalatal elements and tongue retractors that may be implemented in a dental retraction device that eliminate or diminish the disadvantages and problems described above.

The invention is defined in the appended claims.

One aspect of an embodiment includes a tongue retractor. The tongue retractor may be configured to be placed below a tongue of a user and to retain and support the tongue during a dental procedure. The tongue retractor may include a first curved member, a second curved member, a concave surface, and a sublingual buffer. The first curved member and the second curved member (collectively, the curved members) may extend substantially in a transverse direction towards one another to meet at a central region. The first curved member may include a first connection region at a first end. The second curved member may include a second connection region at a second end. The curved members may extend in the longitudinal direction from the connection regions to lowermost regions and then extend in an opposite longitudinal direction from the lowermost regions to the central region. The portions of the curved members between the connection regions to the lowermost regions may be substantially planar. The portions of the curved members between the connection regions and the lowermost regions and the portions of the curved members between the lowermost regions and central region may be configured to engage a floor of a mouth of a user. The curved members may be rotatably flexible at the first connection region and the second connection region. For instance, the curved members may be rotatably flexible such that the central region is rotatable in the longitudinal direction. The curved members may be integrally formed with the concave surface. The central region may be opposite the second connection region on the second curved member and may be opposite the first connection region of the first curved member. The concave surface may extend between forward portions of the curved members. The concave surface may be arced in a longitudinal direction along a rear edge of the concave surface. The concave surface may be further arced in the longitudinal direction between the central region and the rear edge. The sublingual buffer may be located along a part of the forward portions of the curved members. The sublingual buffer may include a buffer thickness that is greater than thicknesses of remaining portions of the forward portions of the curved members. The concave surface may be configured to contact a lower surface of a tongue of a user when placed in a mouth of the user. The sublingual buffer may be comprised at least partially of a first material and the curved members may be comprised at least partially of a second material that is more rigid than the first material.

Another aspect includes a dental retraction device. The dental retraction device may be configured for placement in a mouth of a user during a dental procedure, for instance. The dental retraction device may include a right connection, a left connection, a mandible member, a maxilla member, a tongue retractor, and a sublingual buffer. The left connection may be displaced from the right connection in a transverse direction. The mandible member may be formed with the right connection on a first end and with the left connection on a second end. The mandible member may extend in a forward direction from to the left and right connections. The maxilla member may be formed with the right connection on a first end and with the left connection on a second end. The maxilla member may extend in a longitudinal direction from the left and right connections. The tongue retractor may be formed with the right connection and the left connection. The tongue retractor may be biased at a particular rotational position relative to the mandible member. The tongue retractor may be configured for placement below a tongue of the user to raise a tongue away from the mandible member and towards the maxilla member. The tongue retractor may include curved members and a concave surface. The concave surface may extend between forward portions of curved members. The concave surface may be arced in the longitudinal direction along a rear edge of the concave surface. The concave surface may be further arced in the longitudinal direction between a central region of the concave surface and the rear edge. A first curved member and a second curved member may extend substantially in a transverse direction towards one another to meet at the central region. A portion of the curved members may be substantially planar. The portion of the curved members may be angled in a rearward direction from the central region to the left and right connections to engage a floor of a mouth of a user. An arc along the rear edge may extend forward of the maxilla member. The arc may include a curvature, which may be sufficient to define a volume configured to receive a lower surface of the tongue. The sublingual buffer may be located along a part of the forward portions of the curved members. The sublingual buffer may include a buffer thickness. The buffer thickness may be greater than thicknesses of remaining portions of the forward portions of the curved members.

Another aspect may include a dental retraction device. The dental retraction device may include a right connection, a left connection, a first arced member, a second arced member, and a tongue retractor. The left connection may be displaced from the right connection in a transverse direction. The first arced member may extend between the right connection and the left connection. The second arced member may extend between the right connection and the left connection. The tongue retractor may be at least partially positioned in a volume defined by the first arced member and the second arced member. The tongue retractor may be configured to be placed below a tongue of a user and to retain and support the tongue during a dental procedure. The tongue retractor may include a first curved member, a second curved member, a concave surface, and a sublingual buffer. The first curved member and the second curved member (collectively, the curved members) may extend substantially in a transverse direction towards one another to meet at a central region. The first curved member may include a first connection region at a first end. The second curved member may include a second connection region at a second end. The curved members may extend in the longitudinal direction from the connection regions to lowermost regions and then extend in an opposite longitudinal direction from the lowermost regions to the central region. The portions of the curved members between the connection regions to the lowermost regions may be substantially planar. The portions of the curved members between the connection regions and the lowermost regions and the portions of the curved members between the lowermost regions and central region may be configured to engage a floor of a mouth of a user. The curved members may be rotatably flexible at the first connection region and the second connection region. For instance, the curved members may be rotatably flexible such that the central region is rotatable in the longitudinal direction. The curved members may be integrally formed with the concave surface. The central region may be opposite the second connection region on the second curved member and may be opposite the first connection region of the first curved member. The concave surface may extend between forward portions of the curved members. The concave surface may be arced in a longitudinal direction along a rear edge of the concave surface. The concave surface may be further arced in the longitudinal direction between the central region and the rear edge. The sublingual buffer may be located along a part of the forward portions of the curved members. The sublingual buffer may include a buffer thickness that is greater than thicknesses of remaining portions of the forward portions of the curved members. The concave surface may be configured to contact a lower surface of a tongue of a user when placed in a mouth of the user. The sublingual buffer may be comprised at least partially of a first material and the curved members may be comprised at least partially of a second material that is more rigid than the first material.

Yet another aspect may include a dental retraction device. The dental retraction device may include a first arced member, a second arced member, a tongue retractor, and a sublingual buffer. The first arced member may extend between a right connection and a left connection. The left connection may be displaced from the right connection. For instance, the left connection may be displaced from the right connection in a transverse direction. The second arced member may extend between the right connection and the left connection. The second arced member may be angularly offset relative to the first arced member. The tongue retractor may include a first end, a second end, a first curved member, and a second curved member. The first end may be formed with the left connection. The second end may be formed with the right connection. The first curved member and the second curved member may extend substantially in a transverse direction from the first and second ends to meet at a central region. The concave surface may extend between forward portions of the curved members. The concave surface may be arced in a longitudinal direction along a rear edge of the concave surface. The concave surface may be configured to engage and receive a lower surface of a tongue of a user. The concave surface may include at least a portion of a saddle curve that extends from the curved members to the central region. An arc along the rear edge may be curved in a downward direction towards the curved members. The arc along the rear edge extends forward of the first arced member and includes a curvature sufficient to define a volume configured to receive a lower surface of a tongue. The concave surface may be arced in the longitudinal direction between the central region and the rear edge. The concave surface may be curved in a direction away from the curved members. The sublingual buffer may be located along a part of the forward portions of the curved members. The sublingual buffer may include a buffer thickness. The buffer thickness may be greater than thicknesses of remaining portions of the curved members. A portion of the curved members may be substantially planar. Additionally or alternatively, the portions of the curved members may be angled in a rearward direction from the central region to the left and right connections to engage a floor of a mouth of a user. The portion that is substantially planar may include the portions of the curved members between the right and left connections and lowermost regions. Additionally or alternatively, the portion that is substantially planar may include the portions of the curved members between the lowermost regions and the central region. The tongue retractor may be configured such that the curved members are rotatably flexible relative to the left and right connections. The tongue retractor may be biased at a particular rotational position relative to the second arced member.

A further aspect may include a tongue retractor. The tongue retractor may include one or more components that may include a transpalatal element. The transpalatal element may further include a cradle. The cradle may be positioned between flexible regions. The cradle may include one or more curved members, a concave surface, and a base. The curved members may extend from the flexible regions to an apex. The apex may form an arch-shaped region. The flexible regions may enable movement of the cradle. The flexible regions may enable such movement responsive to a pressure on the tongue retractor such as a downward pressure. The movement may result in the base of the cradle engaging or at least partially contacting a floor of a mouth. Engagement between the base and the floor may stabilize and support a tongue when the tongue engages with the cradle. The concave surface may extend between the curved members across at least a portion of the arch-shaped region. The concave surface may be sized and configured to correspond to a portion of a mouth in which the tongue retractor is used. In detail, the concave surface may be sized and configured to substantially correspond to a shape of an underside of a tongue. The concave surface may be sized and configured such that the tongue is readily received and stabilized in the concave surface or a volume defined by the concave surface. The concave surface may include an upper edge. The upper edge may include a thickness greater than that of the concave surface. The base generally corresponds to a shape of a lower dental arch such that the base fits within bones of the lower dental arch.

These and other aspects, features and advantages of the present invention will become more fully apparent from the following brief description of the drawings, the drawings, the detailed description of preferred embodiments and appended claims.

### BRIEF DESCRIPTION OF THE DRAWINGS

The appended drawings contain figures of preferred embodiments to further illustrate and clarify the above and other aspects, advantages, and features of the present invention. It will be appreciated that these drawings depict only preferred embodiments of the invention and are not intended to limit its scope. The invention will be described and explained with additional specificity and detail through the use of the accompanying drawings in which:
Figure 1A illustrates a first dental retraction device;
Figure 1B illustrates another view of the first dental retraction device;
Figure 1C illustrates another view of the first dental retraction device;
Figure 1D illustrates another view of the first dental retraction device;
Figure 2A illustrates a first example transpalatal element that may be implemented in the first dental retraction device of Figure 1A;
Figure 2B illustrates another view of the first transpalatal element of Figure 2A;
Figure 2C illustrates another view of the first transpalatal element of Figure 2A;
Figure 3 illustrates the first dental retraction device of Figure 1A in an example use arrangement;
Figure 4A illustrates a second dental retraction device;
Figure 4B illustrates another view of the second dental retraction device; and
Figure 5 illustrates the second dental retraction device of Figure 4A in an example use arrangement,
   all in accordance with at least one embodiment described in the present disclosure.

### DETAILED DESCRIPTION OF SOME EXEMPLARY EMBODIMENTS

The present invention is directed towards dental retraction devices that include tongue retractors. The principles of the present disclosure, however, are not limited dental retraction devices. It will be understood that, in light of the present disclosure, the components and arrangements disclosed herein can be successfully used in connection with other types of dental components and/or dental retraction devices.

Additionally, to assist in the description of the dental retraction devices, words such as top, bottom, front, rear, right, and left may be used to describe the accompanying figures. It will be appreciated that the dental retraction devices can be disposed in other positions, used in a variety of situations and may perform a number of different functions. In addition, the drawings may be to scale and may illustrate various configurations, arrangements, aspects, and features of the dental retraction devices. It will be appreciated, however, that the dental retraction devices may have other suitable shapes, sizes, configurations, and arrangements depending, for example, upon the intended use of the dental retraction devices. Further, the dental retraction devices may include any suitable number or combination of aspects, features and the like.

Dental retraction devices may be implemented during dental procedures such as dental restoration procedures. Dental retraction devices are implemented to create a working area (sometimes referred to as a working field) around a tooth or set of teeth on which the procedure is performed. The dental retraction devices separate oral tissue such as the cheeks and/or the tongue from teeth of the user. The dental retraction devices retain the oral tissue in a position away from the teeth and instruments used during dental procedures. Retaining the oral tissue away from the teeth may reduce injury to the patient and reduce interference of the oral tissue with the instruments during the procedure.

Some dental retraction devices are configured for placement in a mouth of the patient. For instance, when in use, all or nearly all (e.g., except one or more lip retainers may extend out and over the lips of the patient) of the dental retraction device may be within the oral cavity. These dental retraction devices may be comprised of an elastically flexible material, which may allow a dental care provider to compress the retraction device. While compressed, the dental care provider may position the retraction device in the oral cavity. The retraction device may then expand in the oral cavity. As the retraction device expands, portions of the retraction devices may be positioned in the patient's mouth to separate the oral tissue from the patient's teeth to create a working area. In particular, the retraction devices may include a first member that is positioned along an upper dental arch of the patient, a second member that is positioned along a lower dental arch of the patient, and a tongue guard that limits movement of the tongue. The first member separates the upper lip and the cheek tissue from the patient's upper teeth. Similarly, the second member separates the lower lip and the cheek tissue from the patient's lower teeth. The tongue guard limits movement of the tongue and retains the tongue away from a working area.

The tongue guard is generally positioned in a central portion of the retraction device. The tongue guard may be connected to the first and second members by a third member. The third member extends from a left side of the retraction device to a right side of the retraction device. For example, when disposed in the patient's oral cavity, the third member extends across the palate of the oral cavity (e.g., from the left side to the right side).

Some conventional tongue guards include a membrane or a surface that is positioned on top of the tongue or have a dome-shape that is positioned over the end of the tongue. These tongue guards cover at least a portion of the tongue or contact a top surface of the tongue. Additionally, these tongue guards press the tongue towards a rear portion of the oral cavity and/or press the tongue towards the inner region of the sublingual floor within the lower dental arch.

This positioning of the tongue introduces some problems. For instance, the tongue guards that press the tongue into the sublingual floor may press the tongue into the saliva glands below the tongue. Such placement of the tongue obstructs access to the saliva glands, which may cause pooling of saliva during the dental procedure. As the saliva accumulates in the oral cavity, the patient can become uncomfortable and the salvia may be introduced into the working area. Additionally, this placement of the tongue does not effectively remove the tongue from a portion of the mouth. Instead, this placement retains and tongue next to many of the teeth that may be involved in the dental procedure. For instance, pressing the tongue into the sublingual floor may interfere with dental procedures being performed on lower molars or lower premolars.

Accordingly, embodiments of the present disclosure include improved dental retraction devices. The dental retraction devices include a tongue retractor that addresses some of the problems of existing retraction devices. For example, the tongue retractor is reshaped and reconfigured to better position the tongue during dental procedures. In general, the tongue retractor is shaped and configured to lift the tongue from the sublingual floor and push the tongue up (e.g., towards the roof of the mouth) and slightly back (towards the throat). This positioning removes the tongue from the sublingual floor and allows access to the saliva glands below the tongue. Furthermore, this positioning retains the tongue in a position away from internal surfaces of the lower teeth, which may improve access to lower teeth such as the molars and premolars.

Lifting the tongue introduces some additional issues. For example, the tissue on the bottom the tongue and the sublingual floor is relatively sensitive to pressure and lifting and pressing the tongue towards the rear portion of the oral cavity can trigger a gag reflex of the patient. Accordingly, the shape and configuration of the retraction device is arranged for comfort of the patient and for a particular position that effectively retains the tongue away from working areas without pressing the tongue into the throat of the patient.

In particular in some embodiments, the tongue retractor includes a transpalatal element, which further includes a cradle. The transpalatal element extends from the left side of the device to the right side of the device, which corresponds to across the palate of a patient. The transpalatal element is flexible or includes flexible regions. The flexible regions allows the cradle to rotate and bend to a comfortable position responsive to weight of the tongue and responsive to a particular geometry of the oral cavity.

The cradle is made up of a curved surface or membrane with multiple radii of curvature. In some embodiments, the cradle creates a "saddle curve." The cradle is accordingly curved to receive and support the bottom surface of the tongue and to lift the tongue from the sublingual floor.

The cradle includes a lower edge. The lower edge is placed against the sublingual floor when the retraction device is in use. The lower edge is shaped and configured for comfort of the patient and to effectively lift the tongue. For instance, the lower edge includes one or more planar portions. The planar portions of the lower edge correspond with relatively flat portions of the sublingual floor. Additionally, the lower edge may also include a sublingual buffer. The sublingual buffer includes a substantially circular structure that extends along the lower edge. The sublingual buffer includes a larger diameter than remaining portions of the cradle, which may better distribute any pressure to the sublingual floor caused by the weight of the tongue placed on the cradle. The sublingual buffer may be constructed of a material or a variation of a material that is soft relative to the material of which the rest of the retraction device is comprised. The material of the sublingual buffer helps distribute any pressure while effectively lifting the tongue from the sublingual floor.

The cradle may also include an upper edge. The upper edge may be in contact with the lower surface of the tongue when the retraction device is in use. For instance, the upper edge may be positioned between the end of the tongue and an end of the sublingual frenulum. The upper edge may be sized and configured to generally direct the tongue towards the roof the mouth and to comfortably retain the tongue in that position during a dental procedure. For example, the upper edge may be concaved in a direction to receive the tongue and support the tongue relative to the sublingual floor. Additionally, the upper edge may have a larger diameter than remaining portions of the cradle. The larger diameter may distribute pressures from the weight of the tongue and reduce a likelihood that the lower surface of the tongue is scratched or injured if the patient moves her tongue.

These and other embodiments are described with reference to the appended drawings in which components and features with common labels indicate like structures and functions unless specified otherwise.

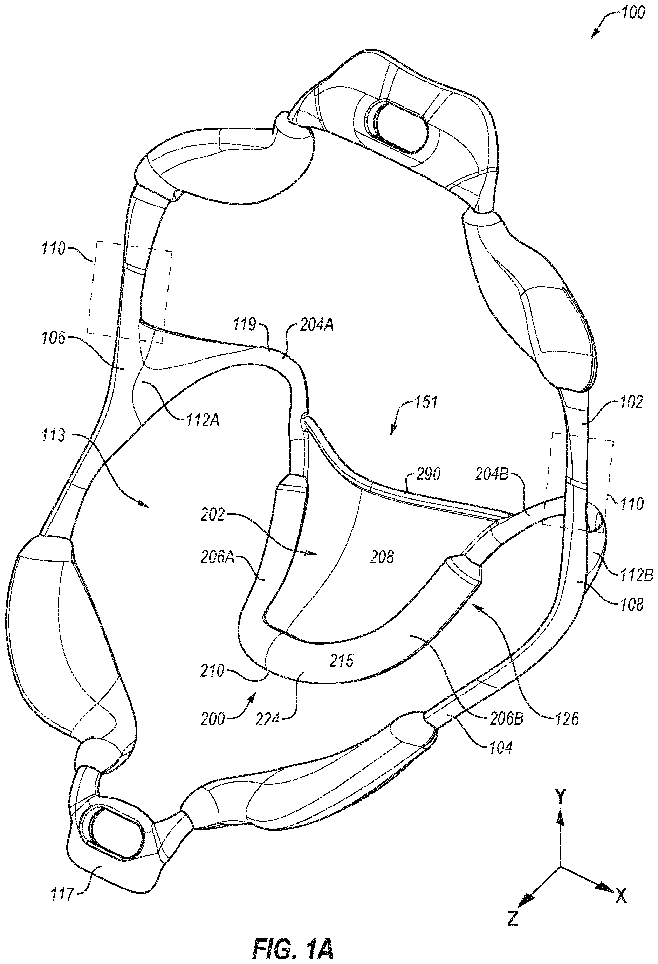

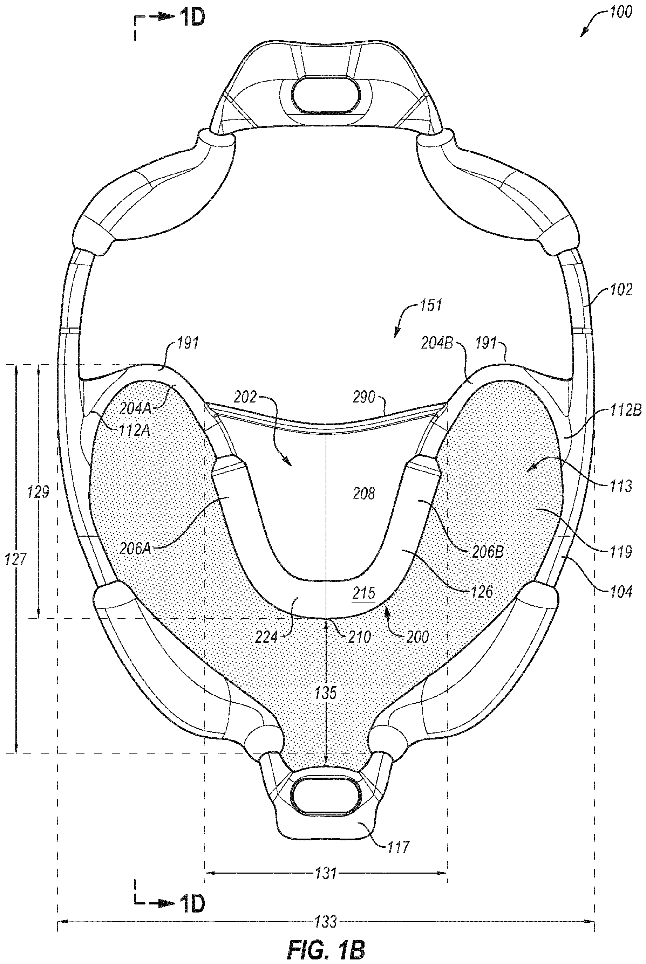

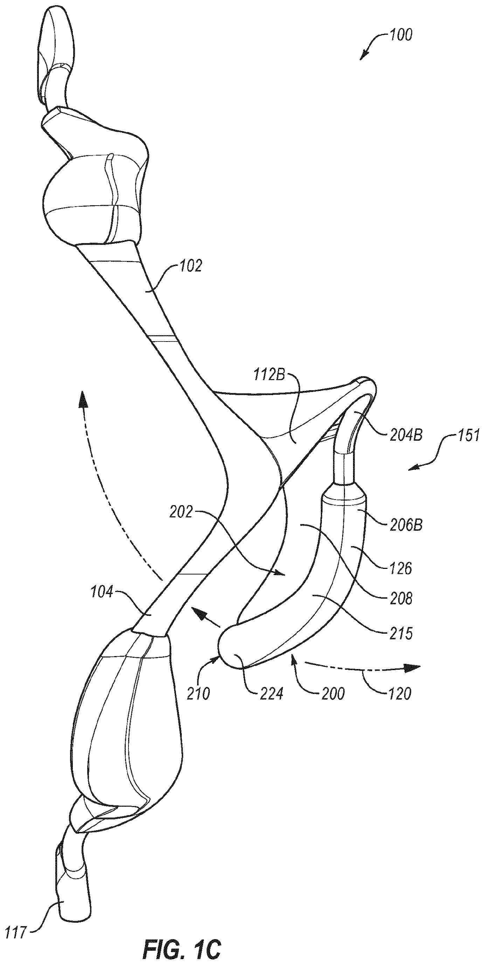

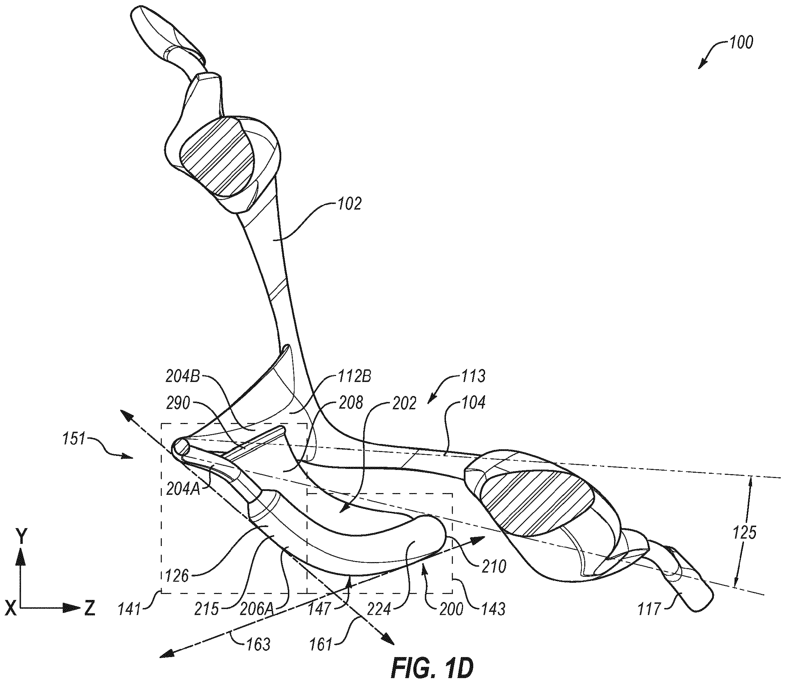

Figures 1A-1D illustrate a first dental retraction device 100 (hereinafter, "first device 100"). Figure 1A is a perspective view of the first device 100. Figure 1B is a front view of the first device 100. Figure 1C is a side view of the first device 100. Figure 1D is a sectional view of the first device 100. The first device 100 is configured to be placed in a mouth of a user such as a patient. For instance, the first device 100 may be placed in the mouth of the user during a dental procedure. When placed in the mouth of the user, the first device 100 may separate lips and cheeks or portions thereof of the user away from outer or external surfaces of the teeth. Additionally, the first device 100 includes a tongue retractor, which is generally identified by 151 in Figures 1A-1D. The tongue retractor 151 may include a first transpalatal element 200. The first transpalatal element 200 is configured to position the tongue away from the teeth of the user. In particular, the first transpalatal element 200 or a portion thereof may be shaped and configured to be placed below the tongue of the user. For instance, the first transpalatal element 200 may contact a portion of the bottom surface of the tongue, which may position the tongue in an upward-displaced arrangement. In the upward-displaced arrangement, the tongue is supported away from the sublingual floor and is in a rear portion of the mouth. In the upward-displaced arrangement, the first device 100 may hold the tongue away from the inner or internal surfaces of the teeth. The first device 100 may accordingly create a work area around the teeth and gums during a procedure.

Positioning the first transpalatal element 200 below the tongue of the user is an improvement relative to tongue retractors that are positioned on an upper surface of the tongue or that include a cavity in which the tongue is placed. In these other tongue retractors, the tongue may be pressed towards the sublingual floor and against the inner or internal surfaces of the teeth or may press the tongue towards the rear portion of the mouth, which may cause discomfort and/or trigger a gag reflex of the user.

The first device 100 of Figures 1A-1D may include a first arced member 102, a second arced member 104, and the tongue retractor 151 that includes the first transpalatal element 200. The first arced member 102, the second arced member 104, and the first transpalatal element 200 may meet and/or be integrally formed with one another at a right connection 106 and a left connection 108. The right connection 106 is separated from or displaced from the left connection 108 in a transverse direction. In Figure 1A, the transverse direction may correspond to the x-direction.

The first device 100 is comprised of a flexible or semi rigid material. The material may be capable of elastic deformation between an expanded arrangement and a compressed arrangement. The expanded arrangement references a configuration of the first device 100 when it is not subject to external forces. For instance, Figure 1A depicts the first device 100 in an expanded arrangement. The compressed arrangement references a configuration of the first device 100 when it is subject to one or more external forces. In the compressed arrangement, the first device 100 may be introduced into a mouth of the user. In some circumstance, in the compressed arrangement the first arced member 102 or some portion thereof may be rotated towards the second arced member 104 and/or the second arced member 104 or some portion thereof may be rotated towards the first arced member 102. Additionally, in some circumstances, in the compressed arrangement the left connection 108 may be displaced generally towards the right connection 106 or vice versa.

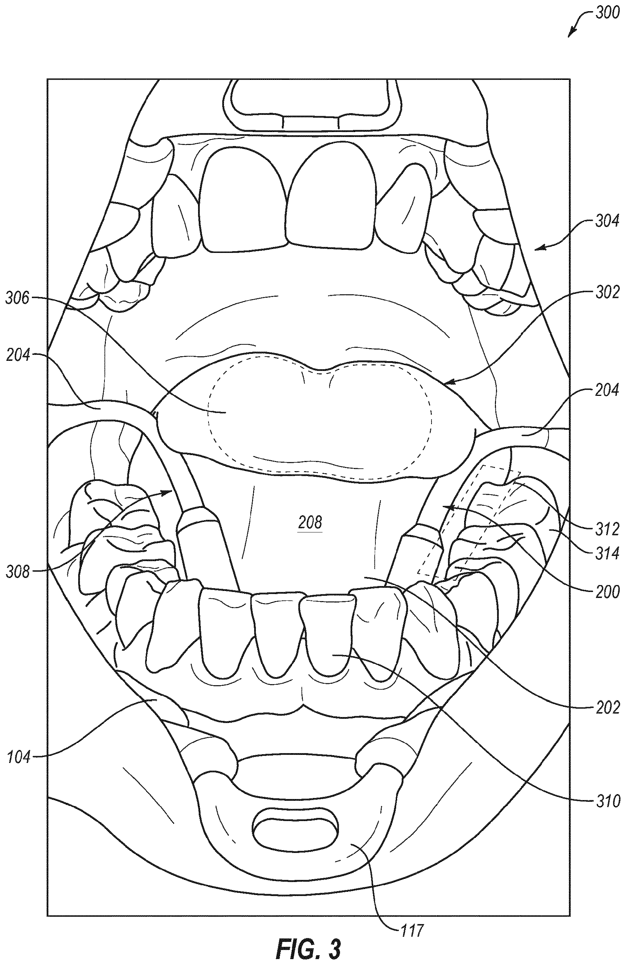

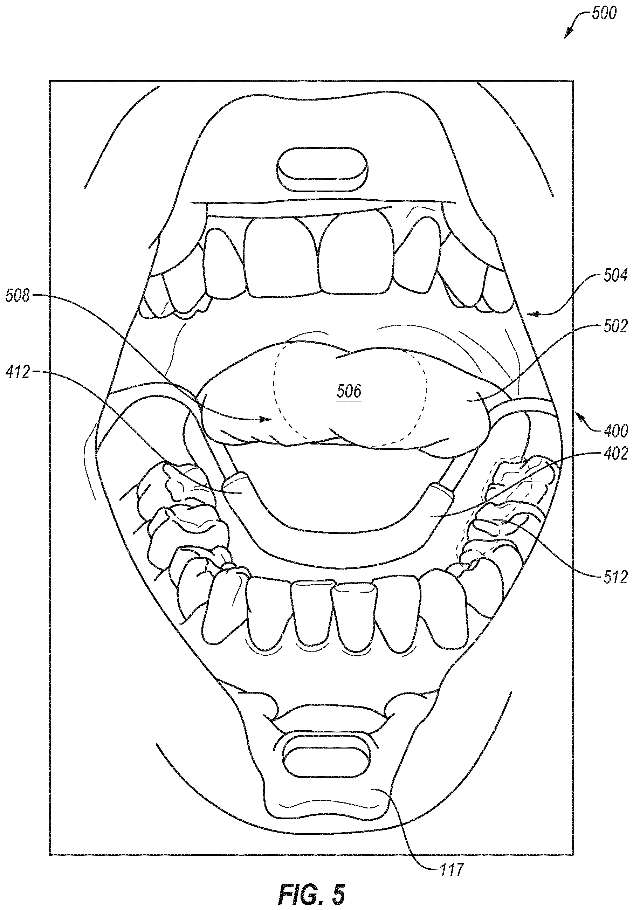

The first device 100 may also be configured in a use configuration. The use configuration references an arrangement when the first device 100 is positioned in the mouth of a user. The use configuration may include geometries that are somewhere between the compressed arrangement and the expanded arrangement. For instance, the first device 100 may be configured in the compressed arrangement. The first device 100 is then placed in the mouth of the user. External forces used to configure the first device 100 in the compressed arrangement may be removed. The first device 100 may then expand to return to the expanded arrangement. However, the first device 100 may be limited by anatomical features of the mouth. Such anatomical features may determine the dimensions of the first device 100 in the use configuration. In the use configuration, the lips and cheeks may be separated from the teeth and the tongue may be positioned in an upward-displaced arrangement. Examples of the use configuration are described with reference to Figures 3 and 5.

The first arced member 102 may extend from the right connection 106 to the left connection 108. The first arced member 102 generally extends in a longitudinal direction, which corresponds to the y-direction of Figure 1A. The second arced member 104 may also extend from the right connection 106 to the left connection 108. When the first device 100 is in an expanded configuration, the second arced member 104 is rotationally offset relative to the first arced member 102. For instance, the second arced member 104 may generally extend in a direction substantially normal (e.g., ± 10 degrees) to the first arced member 102. When the first device 100 is configured in the compressed arrangement, the second arced member 104 or the first arced member 102 may be deformed or bend, such that portions of the second arced member 104 and the first arced member 102 may be rotated towards one another.

In the embodiment of Figures 1A-1D, the first arced member 102 may be configured to be placed along a dental arch of a mandible. The first arced member 102 may be placed along a majority or all (e.g., from a rear-most tooth on a first side to a rear-most tooth on the other side) of the dental arch of the mandible. Accordingly, in the present disclosure, the first arced member 102 is sometimes referred to as a mandible member. The first arced member 102 may include lower regions 110. The lower regions 110 may meet the right connection 106 and the left connection 108. The lower regions 110 may be placed in the mouth of the user next to the upper rearmost molar of the user when the first device 100 is in the use configuration.

Similarly, the second arced member 104 may be configured to be placed along a dental arch of a maxilla. The second arced member 104 may be placed along a majority or an entirety of the dental arch of the maxilla. Accordingly, in the present disclosure, the second arced member 104 is sometimes referred to as a maxilla member. The left and right connections 108 and 106 may be placed in the mouth of the user next to the lower rearmost molar of the user when the first device 100 is in the use configuration.

The first transpalatal element 200 includes a first cradle 202. The first cradle 202 may be at least partially positioned in an inner volume 113 defined by the first arced member 102 and the second arced member 104. The inner volume 113 includes the volume that is generally defined by the first arced member 102 and the second arced member 104 as outer boundaries. For instance, the first cradle 202 may extend in a similar direction to the direction the second arced member 104 extends from the first arced member 102. Additionally, the first cradle 202 may be at least partially positioned within the arched structure formed by the second arced member 104. As discussed elsewhere in the present disclosure, the first cradle 202 may be configured to be placed below a tongue of a user and to retain and support the tongue during a dental procedure. The first cradle 202 may be configured to raise a tongue away from the second arced member 104 and towards the first arced member 102.

The first cradle 202 may include curved members 206A and 206B (generally, curved member 206 or curved members 206). The curved members 206 may be attached to or integrally formed with flexible regions 204A and 204B (generally, flexible region 204 or flexible regions 204). The flexible regions 204 each include an end 112A or 112B. For instance, the first flexible region 204A may include a first end 112A and the second flexible region 204B may include a second end 112B. The first end 112A may be formed (e.g., integrally formed) with the left connection 108. Similarly, the second end 112B may be formed with the right connection 106. Accordingly, the first cradle 202 may be attached to the left and right connections 108 and 106 via the curved members 206 and the flexible regions 204.

In the embodiment of Figures 1A-1D, the first device 100 may be a single piece of material that includes features and components described in the present disclosure. In these and other embodiments, the first end 112A and the second end 112B may be integrally formed as a single piece with the left and right connections 108 and 106, respectively. In other embodiments, the first device 100 may include several pieces or components that are connected or attached to one another. In these embodiments, the first end 112A and the second end 112B may be adhered or otherwise mechanically attached to the left and right connections 108 and 106, respectively.

The flexible regions 204 enable movement of the first cradle 202. For instance, in response to a downward pressure applied by the tongue to the first cradle 202, a base 126 of the first cradle 202 may rotate or move to engage with a sublingual floor. Engagement with the sublingual floor may stabilize and support the tongue. The flexible regions 204 may be rearwardly curved from the left and right connections 108 and 106. For instance, the flexible regions 204 may extend in a direction away from the second arced member 104. The flexible regions 204 may then curve in a forward direction (e.g., towards the second arced member 104). The flexible regions 204 may be formed with the curved members 206.

The curved members 206 may be inwardly curved. The curved members 206 may curve in a transverse direction towards one another. For instance, the first curved member 206A may be curved towards the second curved member 206B in a direction that substantially corresponds to the x-direction of Figure 1A. The first curved member 206A may meet the second curved member 206B at a central region 224, which may include an apex 210. The first cradle 202 may be configured such that the flexible regions 204 are rotatably flexible relative to the left and right connections 108 and 106. For example, with reference to Figure 1C, the first cradle 202 may be configured to rotate along a path labeled 120. Additionally or alternatively, the first cradle 202 may be configured to remain stationary when the first arced member 102 and/or the second arced member 104 are rotated or deformed. For instance, to configure the first device 100 in the compressed arrangement, the second arced member 104 or a portion thereof may be deformed or rotated relative to the first arced member 102. In some embodiments, rotation or deformation of the second arced member 104 relative to the first arced member 102 may not result in rotation of the first cradle 202.

In some embodiments, the first cradle 202 may be biased at a particular rotational position relative to the second arced member 104. For instance, as best illustrated in Figure 1D, the first cradle 202 may be biased at a first angle 125 relative to the second arced member 104. The first angle 125 may be between about 5 degrees and about 25 degrees, between about 10 degrees and about 20 degrees, or between about 12 degrees and about 18 degrees. In some embodiments, as the second arced member 104 rotates or deforms, the first angle 125 may change. For instance, the first cradle 202 may maintain its position and the second arced member 104 or some portion thereof may rotate away from the first cradle 202.

When the first device 100 is in the use arrangement and is placed in a mouth of a user, a base 126 of the first cradle 202 may be positioned against the sublingual floor. The sublingual floor may accordingly push on the base 126. Because the first cradle 202 is biased at the particular rotational position, the first cradle 202 may stay in place below the tongue of the user. Additionally, because the first cradle 202 rotates substantially independently of the second arced member 104, the positioning of the second arced member 104 may not act to alter the position of the first cradle 202. Moreover, if the user closes her mouth (e.g., to take a break during a procedure), the motion of the jaw of the user may not act to alter the position of the first cradle 202. Thus, the tongue of the user may remain engaged with the first cradle 202.

The first cradle 202 includes a first engagement surface 208, which is also referred to as a concave surface. For example, the first engagement surface 208 may be configured to engage with a tongue of the user. The first engagement surface 208 may extend between forward portions (e.g., towards the apex 210) of the curved members 206. In the embodiments of Figures 1A-1D, the first engagement surface 208 may extend across at least a portion of an arch-shaped region formed by the curved members 206 and that includes the apex 210 and the central region 224.

The first engagement surface 208 is sized and configured to substantially correspond to a shape of an underside of a tongue such that the tongue is received and stabilized by the first cradle 202. For example, the first engagement surface 208 includes a rear edge 290. The first engagement surface 208 may be arced in a longitudinal direction along the rear edge 290. For instance, as shown in Figure 1D, the rear edge 290 may be curved towards the first arced member 102. Additionally, an arc along the rear edge 290 may be curved in a downward direction (e.g., negative y-direction) towards the curved members 206. The arc in the longitudinal direction may continue along at least a portion of the first engagement surface 208 from the rear edge 290 to the central region 224. The first engagement surface 208 may also be arced in the longitudinal direction between the central region 224 and the rear edge 290. The first engagement surface 208 may accordingly include at least a portion of a saddle curve that extends from the curved members 206 to the central region 224.

With reference to Figure 1D, the curved members 206 may include a first region 141 and a second region 143. The first region 141 may extend in a negative longitudinal direction, which corresponds to the negative y-direction of Figure 1D. The first region 141 may further extend in a forward direction towards the second arced member 104, which corresponds to the x-direction in Figure 1D. The first region 141 may be positioned between the ends 112 and the second region 143. In some embodiments, a portion of the first region 141 may be substantially planar or planar. In Figure 1D, a first datum 161 depicts an edge of a first plane that may correspond to the planar or substantially planar portion of the first region 141.

The second region 143 may be forward of the first region 141. In the second region 143, the curved members 206 turn in a positive y-direction. The second region 143 may further extend in the forward direction. The second region 143 may include a lowermost region of the curved members 206. The first cradle 202 narrows (decreases z-dimension). The second region 143 may include a bottom surface 147. The bottom surface 147 of the base 126 may be planar or substantially planar. In Figure 1D, a second datum 163 depicts an edge of a second plane that may correspond to the planar or substantially planar portion of the base 126.

The planar or substantially planar portions of the first region 141 and/or the base 126 may be configured to a geometry of an oral cavity. For instance, the base 126 may generally correspond to a shape of a lower dental arch such that the base 126 fits within bone of the lower dental arch. Similarly, the first region 141 may correspond to a shape of the lower dental arch near the rearmost teeth of a user.

The planar portions or substantially planar portions of the curved members 206 may correspond to the sublingual floor. For instance, a part of the sublingual floor on which the base 126 is placed may be planar. Accordingly, the planar portion or substantially planar portions of the curved members 206 may be comfortably placed on the sublingual floor.

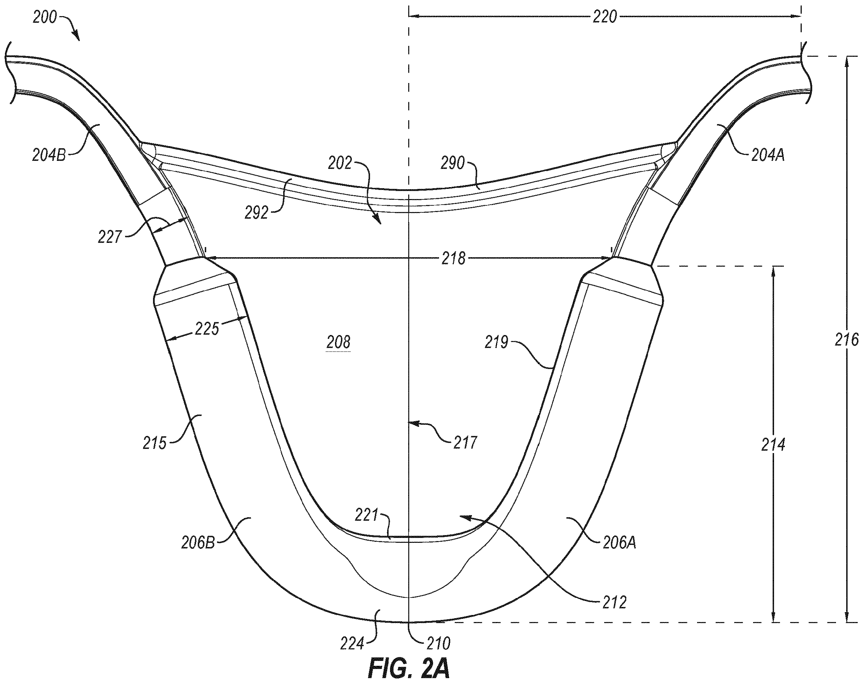

Additionally, the first cradle 202 may be sized relative to the first and second arced members 102 and 104 to be suitable to be positioned below the tongue of the user and against the sublingual floor. For instance, with reference to Figure 1B, the second arced member 104 may include a lip retainer 117. In the use configuration, the lip retainer 117 may be placed on a lower lip of the user. The lip retainer 117 may at least partially extend outside the mouth of the user. The remaining portions of the second arced member 104 may be placed in the mouth and against the lower dental arch. Rearmost portions of the second arced member 104 may include the right and left connections 106 and 108. The curved members 206 may include rearmost portions 191. The rearmost portions 191 may be included on rearward arching portions of the curved members 206. The rearmost portions 191 may be positioned adjacent to and/or behind the rearmost molar or rearmost portion of the lower dental arch of the user. Accordingly, the second arced member 104 and the first cradle 202 may define a curved volume 119 into which the lower teeth of the user may be positioned. The curved volume 119 is depicted in Figure 2A with a hashed pattern. The curved volume 119 may be a part of the inner volume 113. For instance, the curved volume 119 may include the portion of the inner volume 113 defined by the second arced member 104 and the first cradle 202.

The curved volume 119 may include dimensions 127, 129, 131, and 133 that enable the positioning of the second arced member 104 against the lower dental arch and the first cradle 202 below the tongue and against the sublingual floor. For instance, the embodiment of Figure 1B may be suitable for an adult user. The dimensions and values provided below are examples that may be proportionately increased or decreased to accommodate a child or a larger person.

In particular, the first cradle 202 may extend into the curved volume 119. The first cradle 202 may have a cradle length 129 and a cradle width 131. The second arced member 104 may include a member width 133 and a member length 127. The cradle length 129 may be between about 34 and about 46 millimeters, between about 36 mm and about 43 mm, or between about 38 mm and about 40 mm. The member length 127 may be between about 64 and about 76 millimeters, between about 65 mm and about 72 mm, or between about 66 and about 70 mm. Accordingly, the ratio of the member length 127 to the cradle length 129 may be between about 2 to 1 and about 1.6 to 1. In Figure 1B, a distance 135 between the apex 210 and a forward most portion of the curved volume 119 may be between about 25 mm and about 33 mm, about 27 mm and about 31, or between about 28 mm and about 30 mm. As visible in Figure 1B, the cradle width 131 may be between about 31 and about 42 millimeters, between about 33 mm and about 44 mm, or between about 35 and about 37 mm. The member width 133 may be between about 71 and about 84 millimeters, between about 73 mm and about 82 mm, or between about 75 and about 80 mm. Accordingly, the ratio of the member width 133 to the cradle width 131 may be between about 2.3 to 1 and about 1.8 to 1.

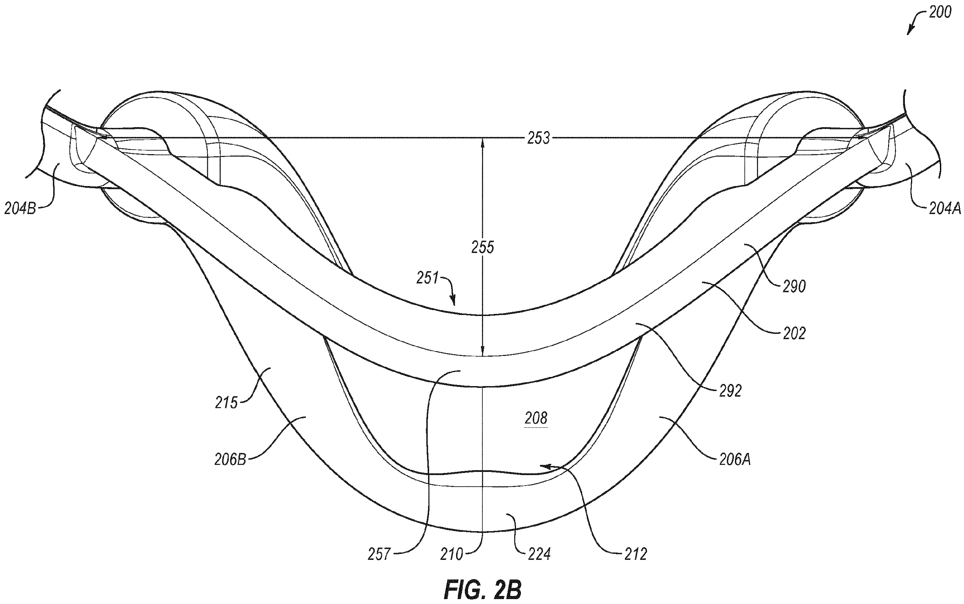

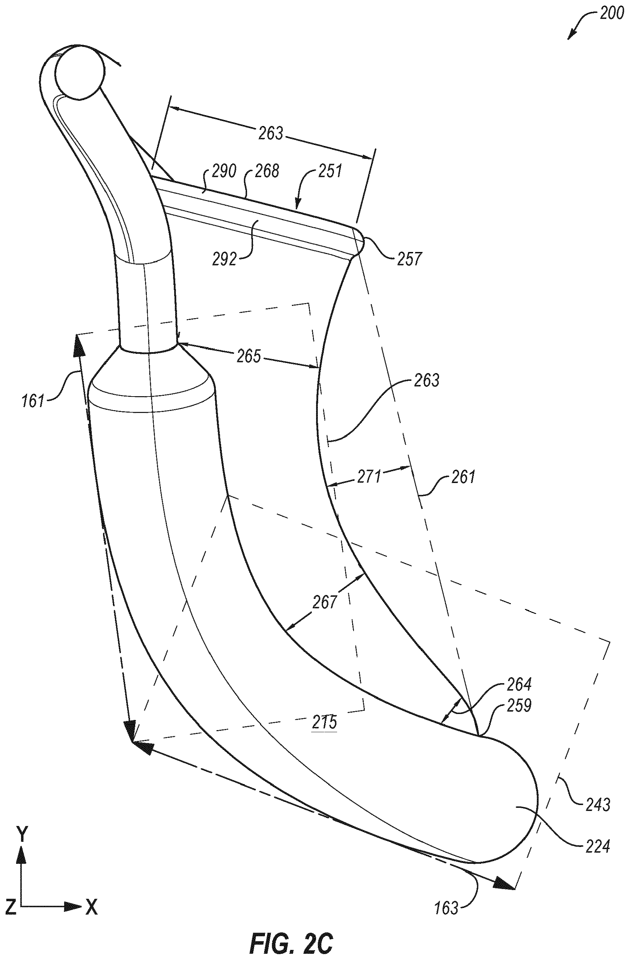

Figures 2A-2C depict an example embodiment of the first transpalatal element 200 that may be implemented in the first device 100 of Figures 1A-1D. Figure 2A depicts a bottom view of the first transpalatal element 200. Figure 2B depicts a top view of the first transpalatal element 200. Figure 2C depicts a side view of the first transpalatal element 200. The first transpalatal element 200 of Figures 2A-2C is configured to be placed below the tongue during use. For instance, during a dental procedure such as a dental restorative procedure or another medical/cosmetic procedure, the first device 100 may be placed in the mouth of a user. Positioning the first device 100 in the mouth includes placement of the first transpalatal element 200 between a lower surface of the tongue and a sublingual surface within the mouth of the user. When placed below the tongue, the first transpalatal element 200 retains and supports the tongue. Accordingly, the tongue may be positioned in an upward-displaced arrangement in which an apex of the tongue is directed towards a roof of the mouth. In the upward-displaced arrangement, the first transpalatal element 200 may separate the tongue from internal surfaces of the teeth in the lower arch, which may reduce or prevent the tongue from interfering with the dental procedure.

The first transpalatal element 200 may include a first cradle 202. The first cradle 202 may be positioned between flexible regions 204A and 204B (generally, flexible region 204 or flexible regions 204). The first cradle 202 may include curved members 206A and 206B (generally curved member 206 or curved members 206) and a first engagement surface 208.

The curved members 206 may extend from the flexible regions 204 to an apex 210. For instance, in Figures 2A-2C, a first curved member 206A extends from a first flexible region 204A and a second curved member 206B extends from a second flexible region 204B. The first curved member 206A meets the second curved member 206B at the apex 210. The curved members 206 form an arch-shaped region, which is generally indicated in Figures 2A-2C at 212.

The curved members 206 may be inwardly curved. For instance, the first curved member 206A and the second curved member 206B curve towards one another in a transverse direction to meet at the central region 224 or the apex 210. Additionally, the portion of the curved members 206 may be angled in a rearward direction from the central region 224 to the left and right connections.

The embodiments of Figures 2A and 2B are defined in the present disclosure in detail. In some embodiments, the first transpalatal element 200 may include another shape. For example, in these and other embodiments, the first transpalatal element 200 may include a shape that is configured to for placement below a tongue of the user and to fit within a dental arch of the user.

With reference to Figure 2C, the curved members 206 may also extend in a longitudinal direction, which may correspond to a negative y-direction of Figure 2C. The curved members 206 may then curve in a positive longitudinal direction, which may correspond to a positive x-direction. One or more portions of the curved members 206 may be substantially planar. For instance, a first substantially planar portion is identified in Figure 2C by a first dashed box 243 and another substantially planar portion is identified in Figure 2C by a second dashed box 263. The portion 243 may include the portion of the curved members 206 near and/or including the central region 224. A lower surface of the portion 243 may be oriented in a plane, which is generally indicated by the datum 163. Similarly, the substantially planar portion 263 may include the portion of the curved members 206 opposite the central region 224. A lower surface of the portion 263 may be oriented in a plane, which is generally indicated by the datum 161.

During use, the curved members 206 may engage a floor of a mouth of a user. For example, at least a portion of the curved members 206 may contact and/or press against the floor of the mouth. When the curved members 206 are engaged with the floor, the overall shape of the curved members 206 may not deform. For instance, the central region 224 may maintain its shape, but the first cradle 202 may rotate at or near regions where the first transpalatal element 200 connects to the flexible regions 204.

Referring to Figures 2A-2C, a sublingual buffer 215 may optionally be located along a part of the forward portions of the curved members 206. The sublingual buffer 215 may include a buffer thickness 225. The buffer thickness 225 may be greater than thickness(es) (e.g., 227 described below) of remaining portions of the curved members 206. For instance, the buffer thickness 225 may be about twice (e.g., between about 1.5 and about 2.5 times) the thickness 227 of the curved members 206. In some embodiments, the sublingual buffer 215 may be comprised of a different material than the curved members 206. For instance, the sublingual buffer 215 may be at least partially of a first material and the curved members 206 may be comprised at least partially of a second material. The second material may be more rigid than the first material. The sublingual buffer 215 may be attached to the curved member 206 to increase comfort when the first transpalatal element 200 is placed in the mouth of the user. For instance, the sublingual buffer 215 may be placed in contact with a lower arch of the user or adjacent to the lower arch of the user.

An arch-shaped region 212 may be sized and configured to match or correspond to a sublingual surface of a mouth of a user. The arch-shaped region 212 may be sized such that the curved members 206 abut or are adjacent to a lower dental arch of a user. For instance, with reference to Figure 2A in some embodiments, which may be implemented in an adult, may include an arch length 214. The arch length 214 may be defined between the apex 210 and an end of the sublingual buffer 215. The arch length 214 may be between about 20 millimeters (mm) and about 30 mm, between about 22 mm and about 29 mm, or about 26 mm and about 28 mm. The arch-shaped region 212 may include an arch width 218. The arch width 218 may be defined between the end of the sublingual buffer 215 and a bisection line 217, which may bisect the first transpalatal element 200. The arch width 218 may be between about 11 mm and 19 mm, between about 13 mm and about 15 mm, or between about 13 mm and about 15 mm. Additionally, between the end of the sublingual buffer 215 and the apex 210, the curved members 206 may curve from a substantially linear portion 219 to a substantially lateral portion 221. The arch-shaped region 212 may include a region length 216. The region length 216 may be defined from an end of the curved member 206 (which may be the rearmost portion of the first device 100) to the apex 210. The region length 216 may be between about 33 mm and about 41 mm, between about 35 mm and about 39 mm, or between about 36 mm and about 37.5 mm. The arch-shaped region 212 may include a region width 220. The region width 220 may be defined from the end of the curved member 206 to the bisection line 217. The region width 220 may be between about 18 mm and about 26 mm, between about 20 mm and about 24 mm, or between about 21 mm and about 22.5 mm.

Referring to Figures 2A-2C, the first engagement surface 208 extends between portions of the curved members 206. For instance, the first engagement surface 208 may extend across at least a portion of the arch-shaped region 212. The first engagement surface 208 is arced to define a volume 251 (Figures 2B and 2C). The volume 251 is shaped and configured to receive a lower surface of the tongue of a user. The first engagement surface 208 may be arced in a longitudinal direction along a rear edge 290 of the first engagement surface 208. The first engagement surface 208 may be further arced in the longitudinal direction between the central region 224 and the rear edge 290. For example, with reference to Figure 2B, a first datum 253 is defined between upper points at which the first engagement surface 208 meets the curved members 206. The first engagement surface 208 curves away from the datum 253. In some embodiments, a distance 255 from the datum 253 to a center point 257 of the first engagement surface 208 may increase between the first curved member 206A and the center point 257. The distance 255 may then decrease between the center point 257 and the second curved member 206B. In some embodiments, the distance 255 may be between about 5 mm and about 10 mm, between about 6 mm and about 9 mm, or about 7 mm and about 8.5 mm. The curvature of the first engagement surface 208 may be similarly curved from the rear edge 290 of the first engagement surface 208 to regions in which the first engagement surface 208 meet the curved members 206.

The first engagement surface 208 also includes a curvature between the curved members 206. For instance, a height of the first engagement surface 208 increases from the curved members 206 to the bisection line 217 such that the bisection line 217 is a peak or approximate peak of the first engagement surface 208. In addition, the height of the first engagement surface 208 above the curved members 206 may generally decrease from the center point 257 to an intersection point 259 where the first engagement surface 208 meets the sublingual buffer 215. For instance, with reference to Figure 2C, a first height 263 may be defined at the center point 257. A second height 265 may be defined at a first distance towards the intersection point 259. A third height 267 may be defined at a second distance towards the intersection point 259. A fourth height 269 may be defined at a third distance towards the intersection point 259. In some embodiments, the first height 263 may be greater than the second height 265, which may be greater than the third height 267, etc. Furthermore, the first engagement surface 208 may be curved in between the center point 257 and the intersection point 259. For instance, a second datum 261 may be defined between the center point 257 and the intersection point 259. The first engagement surface 208 may be curved relative to the second datum 261. In the embodiment of Figure 2C, a distance 271 between the first engagement surface 208 and the second datum 261 may increase from the center point 257 to a region about halfway down the first engagement surface 208. The distance 271 may then decrease between the region and the intersection point 259. The particular distances and dimensions may be suitable for an average adult. These dimensions and distances may be proportionately increased or decreased to accommodate a child, a smaller person, or a larger person.

In some embodiments, the rear edge 290 may include a border 292. The border 292 may include a diameter that is greater than a width of the first engagement surface 208. The border 292 may soften or reduce the sharpness of the rear edge 290. The border 292 may reduce discomfort that may result from the first engagement surface 208 contacting the bottom surface of the tongue during use.

Figure 3 illustrates the first device 100 of Figures 1A-2C in a first example use arrangement 300. Figure 3 depicts the first device 100 positioned in a mouth 304 of the user. In the first use arrangement 300, the first transpalatal element 200 is positioned below a tongue 302. An apex 306 of the tongue 302 is pushed up and back by the first cradle 202. The first engagement surface 208 contacts a bottom surface 308 of the tongue 302. The sublingual buffer 215 or a portion thereof is placed behind lower teeth 310 and against the sublingual floor. The tongue 302 is thus held in an upward-displaced arrangement. The tongue 302 is held back and away from surfaces 312 of lower molars 314. Additionally, the flexible regions 204 are positioned behind the lower molars 314. The second arced member 104 is placed against the lower dental arch to separate cheeks and lips from lower teeth.

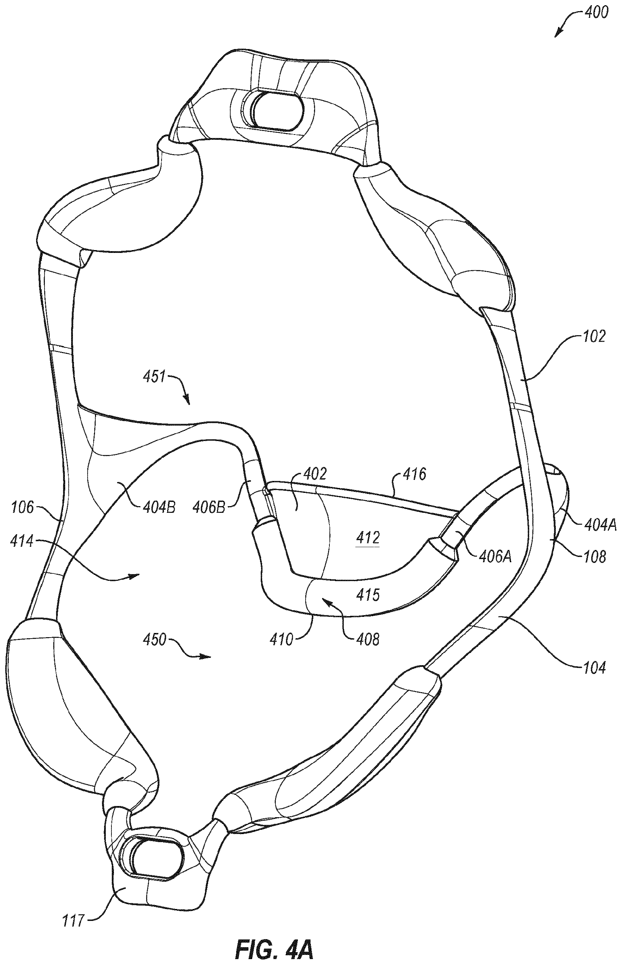

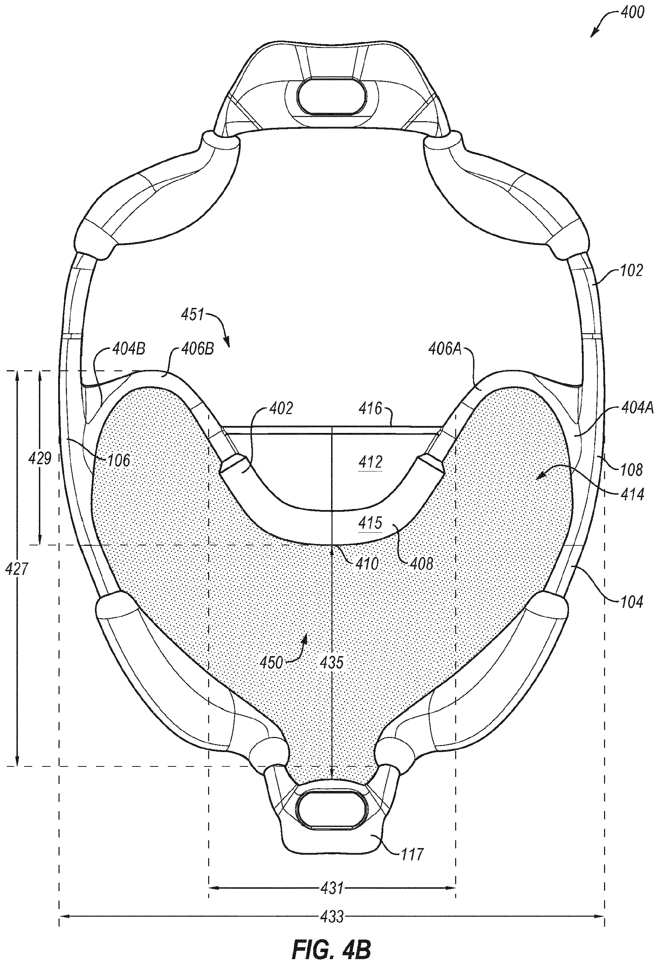

Figures 4A and 4B depict an example embodiment of a second dental retraction device 400 (hereinafter, "second device 400"). Figure 4A is a perspective view of the second device 400. Figure 4B is a front view of the second device 400. The second device 400 is similar to the first device 100. For example, the second device 400 is configured to be placed in a mouth of a user during a dental procedure or another procedure. The second device 400 may accordingly create a work area around the teeth and gums by separating the lips and cheeks of the user away from outer or external surfaces of the teeth.

The second device 400 may include multiple components and features that are included in the first device 100. For example, the second device 400 includes the arced members 102 and 104 that extend between the left connection 108 and the right connection 106. The arced members 102 and 104 are configurable in the compressed arrangement, the use arrangement, and expanded arrangement as described elsewhere in the present disclosure.

The second device 400 includes a second tongue retractor 451 that includes a second transpalatal element 450. The second transpalatal element 450 may be similar to the first transpalatal element 200 described elsewhere in the present disclosure. For instance, the second transpalatal element 450 includes flexible regions 404A and 404B (generally, flexible regions 404 or flexible region 404) that connect to the left connection 108 and the right connection 106, respectively. Like the first transpalatal element 200, the second transpalatal element 450 is configured to be placed below the tongue of a user. For instance, positioning the second device 400 in the mouth may include placement of the second transpalatal element 450 between a lower surface of the tongue and a sublingual surface within the mouth, which may position the tongue in the upward-displaced arrangement. The second transpalatal element 450 may be positioned in a volume 414 that is defined by the arced members 102 and 104.

The second transpalatal element 450 may include a second cradle 402. The second cradle 402 generally extends into the volume 414. The second cradle 402 is similar to the first cradle 202. However, the second cradle 402 includes different dimensions than the first cradle 202. In particular, the second cradle 402 is shorter and smaller than the first cradle 202. For example, the second cradle 402 may include curved members 406A and 406B (generally curved member 406 or curved members 406) that meet at an apex 410 on a central portion 408. The second cradle 402 may be positioned between the flexible regions 404. The flexible regions 404 enable the second cradle 402 to rotate independently or semi-independently of the arced members 102 and 104 like the rotational independence described above with reference to the first device 100.

The second cradle 402 may also include a second engagement surface 412, which may also be a concave surface in some embodiments. The second engagement surface 412 is curved similarly to the first engagement surface 208. For instance, a rear edge 416 may be curved generally in a longitudinal direction towards the first arced member 102. The longitudinal direction may correspond to the y-direction of Figure 4A. The first engagement surface 208 may also curve from the rear edge 416 to the apex 410 and the curved members 406 and in the transverse direction between the flexible regions 404.

Portions of the curved members 406 may be covered by a sublingual buffer 415, which is similar to the sublingual buffer 215. For instance, the sublingual buffer 415 includes a thickness that is greater than remaining portions of the curved members 406. The sublingual buffer 415 may increase the comfort during use by decreasing pressure against a sublingual floor.

The curved members 406 may include the rearmost portions 191 described elsewhere in the present disclosure. Accordingly, the second arced member 104 and the second cradle 402 may define a second curved volume 414 into which lower teeth of the user may be positioned. The second curved volume 414 is greater than the curved volume 119. The second curved volume 414 is depicted in Figure 4B with a hashed pattern. The second curved volume 414 may include dimensions 427, 429, 431, and 433 that enable the positioning of the second arced member 104 against the lower dental arch and the first cradle 202 below the tongue and against the sublingual floor. For instance, the embodiment of Figure 4B may be suitable for an adult user. The dimensions and values provided below are examples that may be proportionately increased or decreased to accommodate a child or a larger person.

In particular, the second cradle 402 may extend into the curved volume 414. The second cradle 402 may have a cradle length 429 and a cradle width 431. The second arced member 104 may include a member width 433 and a member length 427. The member length 427 and the member width 433 may be substantially similar to the member length 127 of Figure 1B and the member width 133 of Figure 1B, respectively. Additionally, the cradle width 431 may be substantially similar to the cradle width 131 of Figure 1B. The cradle length 429 of Figure 4B may be less than the cradle length 129. For instance, the cradle length 429 may be between about 50% and about 70% of the cradle length 129. Accordingly, a distance 435 between the apex 410 and a forward most portion of the second curved volume 414 may be greater than the distance 135 of Figure 1B.

Figure 5 illustrates the second device 400 of Figures 4A and 4B in a second example use arrangement 500. Figure 5 depicts the second device 400 positioned in a mouth 504 of a user. In the second use arrangement 500, the second transpalatal element 450 is positioned below a tongue 502. An apex 506 of the tongue 502 is pushed up (in a positive y-direction) and back (e.g., away from the lips of the user) by the second cradle 402. The second engagement surface 412 contacts a portion of a bottom surface 508 of the tongue 502. The sublingual buffer 415 or a portion thereof is placed against the sublingual floor of the user. The second device 400 is positioned away from inner surfaces 512 of the lower, front teeth 518. For instance, with reference to Figures 3 and 5, the first device 100 in the first use arrangement 300 positions the sublingual buffer 215 in contact with or adjacent to gums or tissue attaching the lower teeth 310. In contrast, the sublingual buffer 415 of the second device 400 is positioned away from the gums and tissue attaching the lower front teeth 518. Instead, the sublingual buffer 415 is positioned in a rear portion of the mouth 504.

The second device 400 supports the tongue 502 back and away from inner surfaces 512 of lower molars 514. Additionally, the flexible regions 404 are positioned behind the lower molars 514. The second arced member 104 is placed against the lower dental arch to separate cheeks and lips from lower teeth. The tongue 502 is thus held in an upward-displaced arrangement and the second device 400 creates a work area for a dental procedure.

By the term "substantially" it is meant that the recited characteristic, parameter, or value need not be achieved exactly, but that deviations or variations, including for example, tolerances, measurement error, measurement accuracy limitations and other factors known to skill in the art, may occur in amounts that do not preclude the effect the characteristic was intended to provide.

Although this invention has been described in terms of certain preferred embodiments, other embodiments apparent to those of ordinary skill in the art are also within the scope of this invention. Accordingly, the scope of the invention is intended to be defined only by the claims which follow.

## Claims

1. A tongue retractor (151) that is configured to be placed below a tongue of a user and lift the tongue from the sublingual floor towards a roof of a mouth of the user, the tongue retractor comprising:
a first curved member (206A);
a second curved member (206B), the curved members extending substantially in a transverse direction towards one another to meet at a central region (224);
a concave surface (208) configured to contact a lower surface of a tongue of a user when placed in a mouth of a user, the concave surface (208) extending between forward portions of the curved members, the concave surface being arced in a longitudinal direction along a rear edge (290,416) of the concave surface and the concave surface is further arced in the longitudinal direction between the central region (224) and the rear edge; and
a sublingual buffer (215,415) located along a part of the forward portions of the curved members, the sublingual buffer including a buffer thickness that is greater than thicknesses of remaining portions of the forward portions of the curved members, the sublingual buffer placed in contact with or adjacent to a lower dental arch of the user to distribute pressure to the sublingual floor of the user while lifting the tongue of the user from the sublingual floor.

2. The tongue retractor of claim 1, wherein:
the first curved member includes a first end;
the second curved member includes a second end;
the central region is opposite the second end of the second curved member and is opposite the first end of the first curved member; and
the curved members extend in the longitudinal direction from the first end and the second end to lowermost regions and then extend in an opposite longitudinal direction from the lowermost regions to the central region.

3. The tongue retractor of claim 2, wherein:
the first curved member includes a connection region at the first end;
the second curved member includes a connection region at the second end;
portions of the curved members between connection regions to the lowermost regions are substantially planar; and
portions of the curved members between the lowermost regions and central region are substantially planar,
optionally wherein:
the portions of the curved members between the connection regions and the lowermost regions and the portions of the curved members between the lowermost regions and central region are configured to engage a floor of a mouth of a user.

4. The tongue retractor of claim 2, wherein the curved members are rotatably flexible at a first connection region and a second connection region such that the central region is rotatable in the longitudinal direction.

5. The tongue retractor of any preceding claim, wherein curved members are integrally formed with the concave surface.

6. The tongue retractor of any preceding claim, wherein the sublingual buffer is comprised at least partially of a first material and the curved members are comprised at least partially of a second material that is more rigid than the first material.

7. A dental retraction device, comprising:
a right connection;
a left connection displaced from the right connection in the transverse direction;
a first arced member that extends between the right connection and the left connection;
a second arced member that extends between the right connection and the left connection; and
the tongue retractor of any one of claims 1 to 6 that is at least partially positioned in a volume defined by the first arced member and the second arced member.

## Patentansprüche

1. Zungenspanner (151), der konfiguriert ist, unter eine Zunge eines Benutzers platziert zu werden und die Zunge vom Sublingualboden in Richtung des Gaumendachs des Benutzers anzuheben, wobei der Zungenspanner enthält:
ein erstes gekrümmtes Element (206A);
ein zweites gekrümmtes Element (206B), wobei sich die gekrümmten Elemente im Wesentlichen in einer Querrichtung zueinander erstrecken, dass sie sich in einem zentralen Gebiet (224) treffen;
eine konkave Oberfläche (208), die konfiguriert ist, dass sie eine untere Oberfläche einer Zunge eines Benutzers berührt, wenn sie in einem Mund eines Benutzers platziert ist, wobei sich die konkave Oberfläche (208) zwischen vorne liegenden Bereichen der gekrümmten Elemente erstreckt, wobei die konkave Oberfläche in einer Längsrichtung entlang einer Hinterkante (290, 416) der konkaven Oberfläche bogenförmig ist und die konkave Oberfläche weiter in der Längsrichtung zwischen dem zentralen Gebiet (224) und der hinteren Kante bogenförmig ist; und
einen Sublingualpuffer (215, 415), der entlang eines Teils der vorne liegenden Bereiche der gekrümmten Elemente sich befindet, wobei der Sublingualpuffer eine Pufferdicke hat, die größer als die Dicke der anderen Bereiche der vorne liegenden Bereiche der gekrümmten Elemente ist, wobei der Sublingualpuffer in Berührung mit einem oder angrenzend an einen unteren Zahnbogen des Benutzers platziert wird, dass der Druck auf den Sublingualboden des Benutzers verteilt wird und gleichzeitig die Zunge des Benutzers vom Sublingualboden angehoben wird.

2. Zungenspanner nach Anspruch 1, wobei:
das erste gekrümmte Element ein erstes Ende enthält;
das zweite gekrümmte Element ein zweites Ende enthält;
das zentrale Gebiet gegenüber dem zweiten Ende des zweiten gekrümmten Elements ist und gegenüber dem ersten Ende des ersten gekrümmten Elements ist; und
die gekrümmten Elemente sich in der Längsrichtung von dem ersten Ende und dem zweiten Ende zu am weitesten unten liegenden Gebieten erstrecken und sich dann in einer gegenüberliegenden Längsrichtung von den am weitesten unten liegenden Gebieten zu dem zentralen Gebiet erstrecken.

3. Zungenspanner nach Anspruch 2, wobei:
das erste gekrümmte Element ein Verbindungsgebiet an dem ersten Ende enthält;
das zweite gekrümmte Element ein Verbindungsgebiet an dem zweiten Ende enthält;
Bereiche der gekrümmten Elemente zwischen den Verbindungsgebieten zu den am weitesten unten liegenden Gebieten im Wesentlichen planar sind; und
Bereiche der gekrümmten Elemente zwischen den am weitesten unten liegenden Gebieten und dem zentralen Gebiet im Wesentlichen planar sind;
wobei optional:
die Bereiche der gekrümmten Elemente zwischen den Verbindungsgebieten und den am weitesten unten liegenden Gebieten und die Bereiche der gekrümmten Elemente zwischen den am weitesten unten liegenden Gebieten und dem zentralen Gebiet konfiguriert sind, dass sie den Mundboden eines Benutzers einnehmen.

4. Zungenspanner nach Anspruch 2, wobei die gekrümmten Elemente an einem ersten Verbindungsgebiet und einem zweiten Verbindungsgebiet flexibel drehbar sind, so dass das zentrale Gebiet in der Längsrichtung drehbar ist.

5. Zungenspanner nach einem der vorhergehenden Ansprüche, wobei die gekrümmten Elemente integral mit der konkaven Oberfläche geformt sind.

6. Zungenspanner nach einem der vorhergehenden Ansprüche, wobei der Sublingualpuffer zumindest teilweise aus einem ersten Material gebildet ist und die gekrümmten Elemente zumindest teilweise aus einem zweiten Material gebildet sind, das steifer als das erste Material ist.

7. Zahnärztliche Retraktionsvorrichtung, enthaltend:
eine rechte Verbindung;
eine linke Verbindung, die von der rechten Verbindung in der Querrichtung beabstandet ist;
ein erstes bogenförmiges Element, das sich zwischen der rechten Verbindung und der linken Verbindung erstreckt;
ein zweites bogenförmiges Element, das sich zwischen der rechten Verbindung und der linken Verbindung erstreckt; und
den Zungenspanner nach einem der Ansprüche 1 bis 6, der zumindest teilweise in einem Volumen positioniert ist, das durch das erste bogenförmige Element und das zweite bogenförmige Element definiert ist.

## Revendications

1. Écarteur de langue (151) configuré pour être positionné sous une langue d'un utilisateur et pour soulever la langue du fond sublingual en direction de la voûte palatine de l'utilisateur, l'écarteur de langue comprenant :
un premier organe courbe (206A) ;
un deuxième organe courbe (206B),
les organes courbes s'étendant sensiblement selon une direction transversale l'un vers l'autre pour se rencontrer dans une zone centrale (224) ;
une surface concave (208) configurée pour venir au contact d'une surface inférieure d'une langue d'un utilisateur lorsqu'elle est placée dans une bouche d'un utilisateur, la surface concave (208) s'étendant entre des parties antérieures des organes courbes, la surface concave étant en forme d'arc selon une direction longitudinale le long d'un bord postérieur (290, 416) de la surface concave et la surface concave est en outre en forme d'arc selon la direction longitudinale entre la zone centrale (224) et le bord postérieur ; et
un tampon sublingual (215, 415) situé le long d'une partie des parties antérieures des organes courbes, le tampon sublingual comportant une épaisseur de tampon supérieure aux épaisseurs des parties restantes des parties antérieures des organes courbes, le tampon sublingual étant placé au contact de, ou adjacent à, un arc dentaire inférieur de l'utilisateur afin de distribuer la pression sur le fond sublingual de l'utilisateur tout en soulevant la langue de l'utilisateur du fond sublingual.

2. Écarteur de langue selon la revendication 1, selon lequel :
le premier organe courbe comporte une première extrémité ;
le deuxième organe courbe comporte une deuxième extrémité ;
la zone centrale est à l'opposé de la deuxième extrémité du deuxième organe courbe et est à l'opposé de la première extrémité du premier organe courbe ; et
les organes courbes s'étendent selon la direction longitudinale depuis la première extrémité et la deuxième extrémité vers des zones les plus inférieures et puis s'étendent selon une direction longitudinale opposée depuis les zones les plus inférieures vers la zone centrale.

3. Écarteur de langue selon la revendication 2, selon lequel :
le premier organe courbe comporte une zone à connexion à la première extrémité ;
le deuxième organe courbe comporte une zone à connexion à la deuxième extrémité ;
les parties des organes courbes entre les zones à connexion et les zones les plus inférieures sont sensiblement planes ; et
les parties des organes courbes entre les zones les plus inférieures et la zone centrale sont sensiblement planes,
optionnellement selon lequel :
les parties des organes courbes entre les zones à connexion et les zones les plus inférieures et les parties des organes courbes entre les zones les plus inférieures et la zone centrale sont configurées pour s'engager avec un fond buccal d'un utilisateur.

4. Écarteur de langue selon la revendication 2, selon lequel les organes courbes sont flexibles en rotation au niveau d'une première zone à connexion et au niveau d'une deuxième zone à connexion, de sorte que la zone centrale est capable de rotation selon la direction longitudinale.

5. Écarteur de langue selon l'une quelconque des revendications précédentes, selon lequel les organes courbes sont formés de manière intégrale avec la surface concave.

6. Écarteur de langue selon l'une quelconque des revendications précédentes, selon lequel le tampon sublingual est constitué au moins partiellement d'un premier matériau et les organes courbes sont constitués au moins partiellement d'un deuxième matériau qui est plus rigide que le premier matériau.

7. Dispositif écarteur dentaire, comprenant :
une connexion de droite ;
une connexion de gauche, déplacée par rapport à la connexion de droite selon une direction transversale ;
un premier organe en forme d'arc s'étendant entre la connexion de droite et la connexion de gauche ;
un deuxième organe en forme d'arc s'étendant entre la connexion de droite et la connexion de gauche ; et
l'écarteur de langue selon l'une quelconque des revendications 1 à 6, qui est positionné au moins partiellement dans un volume défini par le premier organe en forme d'arc et le deuxième organe en forme d'arc.
